# EUROPEAN PATENT APPLICATION

(11) **EP 3 955 174 A2**
(43) Date of publication of application: **16.02.2022**
(21) Application number: 21202496.2
(22) Date of filing: 13.10.2021
(51) Int. Cl.: G06N 3/08, G06F 9/50

(54) **METHOD, APPARATUS AND STORAGE MEDIUM FOR TRAINING A DEEP LEARNING FRAMEWORK**

(30) Priority: 10.03.2021 CN 202110266288
(71) Applicant: BEIJING BAIDU NETCOM SCIENCE AND TECHNOLOGY CO. LTD., 100085 Beijing (CN)
(72) Inventor: He, Tianjian, Beijing, 100085 (CN); Yu, Dianhai, Beijing, 100085 (CN); Wu, Zhihua, Beijing, 100085 (CN); Dong, Daxiang, Beijing, 100085 (CN); Ma, Yanjun, Beijing, 100085 (CN)
(74) Representative: advotec.

(57) **Abstract**

The present disclosure discloses a method, an apparatus and a storage medium for training a deep learning framework, and relates to the artificial intelligence field such as deep learning and big data processing. The specific implementation solution is: acquiring at least one task node in a current task node cluster, that meets a preset opening condition when a target task meets a training start condition; judging whether a number of nodes of the at least one task node is greater than or equal to a preset number; synchronously training the deep learning framework of the target task by the at least one task node according to sample data if the number of nodes is greater than the preset number; and acquiring a synchronously trained target deep learning framework when the target task meets a training completion condition.

## Description

### TECHNICAL FIELD

The present disclosure relates to technical fields such as deep learning and big data processing, and in particular to a method, an apparatus and a storage medium for training a deep learning framework.

### BACKGROUND

With the application of computer technology, deep learning technology has achieved great success in the field of supervised learning, wherein in the training of deep learning model frameworks, in order to solve the problem of the costly acquiring of large-scale labeled data, unsupervised technology is gradually being valued by people. Among many unsupervised learning techniques, comparative learning has shown extraordinary potential in the past year, and indicators on many tasks can even approach the effects of supervised learning already. Specifically, in the field of vision, training of a deep model framework expects to learn a representation of an image through certain pre-training tasks, and then the learned representation will be applied directly to specific tasks.

However, in actual training, tasks will run by submitting cluster jobs, and multiple people share a batch of machine resources. If pre-training tasks occupy cluster resources for a long time, research tasks of other users or some tasks of high priority would be caused to fail to execute. Moreover, even if our task is successfully submitted, we must frequently observe whether the execution fails. If it is found that the task fails, it needs to manually re-submit the task, which causes the training very inefficient.

### SUMMARY

The present disclosure provides a method, an apparatus and a storage medium for training a deep learning framework, for reducing manpower investment for task training in a cluster and improving training efficiency.

According to one aspect of the present disclosure, a method for training a deep learning framework is provided, which includes: acquiring at least one task node in a current task node cluster, that meets a preset opening condition when a target task meets a training start condition; judging whether a number of nodes of the at least one task node is greater than a preset number; synchronously training the deep learning framework of the target task by the at least one task node according to sample data when the number of nodes is greater than the preset number; and acquiring a synchronously trained target deep learning framework when the target task meets a training completion condition.

According to another aspect of the present disclosure, an apparatus for training a deep learning framework is provided, which includes: a first acquiring module, configured to acquire at least one task node in a current task node cluster, that meets a preset opening condition when a target task meets a training start condition; a judging module, configured to judge whether a number of nodes of the at least one task node is greater than a preset number; a training module, configured to synchronously train the deep learning framework of the target task by the at least one task node according to sample data when the number of nodes is greater than the preset number; and a second acquiring module, configured to acquire a synchronously trained target deep learning framework when the target task meets a training completion condition.

According to further another aspect of the present disclosure, an electronic device is provided, which includes: at least one processor; and a memory communicatively connected with the at least one processor; wherein instructions executable by the at least one processor are stored in the memory, and the instructions are executed by the at least one processor, so that the at least one processor can execute the method for training a deep learning framework according to the embodiment of the first aspect.

According to yet another aspect of the present disclosure, a non-transitory computer-readable storage medium is provided, in which computer instructions are stored, wherein the computer instructions are configured to cause the computer execute the method for training a deep learning framework according to the embodiment of the first aspect.

According to still another aspect of the present disclosure, a computer program product, comprising a computer program that, when executed by a processor, implements the method for training a deep learning framework according to the embodiment of the first aspect.

The embodiments of the present disclosure have at least the following technical effects:
at least one task node in a current task node cluster is acquired, which meets a preset opening condition when a target task meets a training start condition, and then it is judged whether the number of nodes of the at least one task node is greater than a preset number, if the number of nodes is greater than the preset number, the deep learning framework of the target task is synchronously trained according to the at least one task node according to sample data; at last a synchronously trained target deep learning framework is acquired when the target task meets a training completion condition. As a result, automatic and flexible training of the deep learning framework is realized, and under the premise of ensuring the training effect, the training efficiency is improved and the manpower cost is reduced.

It should be understood that the content described in the present section is not intended to identify the key or important features of the embodiments of the present disclosure, nor is it intended to limit the scope of the present disclosure. Other features of the present disclosure will be easily understood through the following description.

### BRIEF DESCRIPTION OF THE DRAWINGS

The drawings are used to better understand the present solution, and do not constitute a limitation to the present disclosure, in which:
Fig. 1 is a schematic flowchart of a method for training a deep learning framework according to a first embodiment of the present disclosure;
Fig. 2 is a schematic diagram of a state of target task training according to a second embodiment of the present disclosure
Fig. 3 is a schematic flowchart of a method for training a deep learning framework according to a third embodiment of the present disclosure;
Fig. 4 is a schematic flowchart of a method for training a deep learning framework according to a fourth embodiment of the present disclosure;
Fig. 5 is a schematic structural diagram of an apparatus for training a deep learning framework according to a fifth embodiment of the present disclosure;
Fig. 6 is a schematic structural diagram of an apparatus for training a deep learning framework according to a sixth embodiment of the present disclosure;
Fig. 7 is a block diagram of an electronic device used to implement the method for training a deep learning framework according to an embodiment of the present disclosure.

### DETAILED DESCRIPTION

The following describes exemplary embodiments of the present disclosure with reference to the accompanying drawings, which include various details of the embodiments of the present disclosure to facilitate understanding, and should be regarded as merely exemplary. Therefore, those of ordinary skill in the art should recognize that various changes and modifications can be made to the embodiments described herein without departing from the scope of the present disclosure. Likewise, for clarity and conciseness, descriptions of well-known functions and structures are omitted in the following description.

In order to solve the problem mentioned in the above background technology that, when a deep learning framework is trained in cluster resources, manual monitoring of the training status is required, which leads to high manpower costs, and training tasks may affect other tasks and occupy cluster resources for a long time, resulting in tasks of high priority cannot be executed, the present disclosure proposes a method for flexible training a deep learning framework for a deep learning model. In the case that the cluster resources are idle, the execution of our pre-training tasks is automatically performed without human monitoring. And our tasks will be killed when there are tasks of higher priority to avoid affecting the execution of other tasks of high priority.

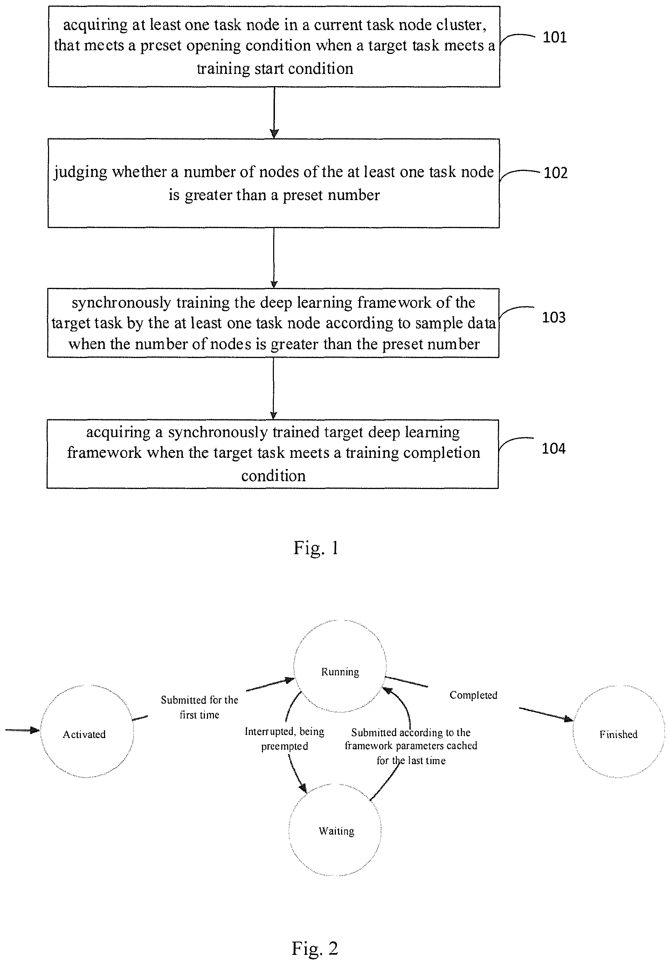

Specifically, Fig. 1 is a flowchart of a method for training a deep learning framework according to an embodiment of the present disclosure. As shown in Fig. 1, the method includes:
a step 101 of acquiring at least one task node in a current task node cluster, that meets a preset opening condition when a target task meets a training start condition.

Wherein the target task may be any task used for training the deep learning framework by deep learning, including recognition of image categories, label classification of medicines, semantic recognition of images and the like.

For the current task node cluster, it is not difficult to understand that the cluster resources may carry a large number of training tasks, and the execution of the training of the target task requires a certain amount of resources. Therefore, in order to ensure that the target task can be executed, at least one task node in a current task node cluster will be acquired, that meets a preset opening condition when a target task meets a training start condition, wherein each node in the cluster may be understood as a machine resource, which can be used for the training of the deep learning framework according to the sample data.
a step 102 of judging whether the number of nodes of the at least one task node is greater than a preset number.

Wherein the preset number may be determined according to the amount of the training task of the target task. In some possible embodiments, a deep learning model can be obtained in advance by training according to experimental data, and the input of the deep learning model is the task type of the target task, and the output is the preset number, so that the corresponding task type can be obtained according to the deep learning model.

It should be understood that when there are fewer task nodes, it is obviously unable to carry the training intensity of the target task. Therefore, it is judged whether the number of the nodes of the at least one task node is greater than or equal to the preset number.
a step 103 of synchronously training the deep learning framework of the target task by the at least one task node according to sample data if the number of nodes is greater than the preset number.

In the present embodiment, if it is greater than the preset number, it indicates that the target task can be started, and the deep learning framework of the task is synchronously trained according to all task nodes that meet the condition.

Wherein the deep learning framework may be the initial architecture of any form of deep learning model, which may include a convolutional layer, a pooling layer, a fully connected layer, and so on.

It should be understood that acquisition channels and acquisition time and the like of the sample data of different nodes may be different. Therefore, the sample data used by different task nodes during training may lead to different training results. Therefore, in order to ensure the comprehensiveness of the training effect, in an embodiment of the present disclosure, the deep learning framework of the task will be trained synchronously according to all task nodes that meet the condition, that is, the training results of all task nodes will be synchronized.

In one embodiment of the present disclosure, the deep learning framework will be trained in each task node, and the framework parameters of the deep learning framework in each task node in each period will be read according to a preset period. The framework parameters may be understood as the model parameters of the deep learning model, etc. And a first average value will be determined, which is the average value of the framework parameters of all task nodes, and the deep learning framework in each task node will be synchronized according to the first average value, thus the synchronization of training between different task nodes is realized.

In another embodiment of the present disclosure, the deep learning framework is divided into multiple training stages, and when each training stage is reached, the framework parameters for the training stage in each task node will be acquired, and the synchronization of each training stage will be realized after the frame parameters of the stage are averaged.
a step 104 of acquiring a target deep learning framework for synchronous training when the target task meets a training completion condition.

Wherein in some possible embodiments, if a training task completion condition sent by a user is received, the target task is considered to meet the training completion condition; in other possible embodiments, in each task node, according to a loss function, a loss value of the deep learning framework will be calculated. When the loss value of all task nodes is less than a preset threshold, it is considered that the target task meets the training completion condition.

When the target task meets the training completion condition, since all task nodes are trained synchronously, the synchronously trained target deep learning framework of all task nodes will be acquired, and the target deep learning framework is the training result of all task nodes, which are synchronized, and then according to the target deep learning framework, any scenario, which requires the target deep learning framework, will be performed, such as semantic recognition of images through the target deep learning framework; such as image classification through the target deep learning framework; such as determination of labels of medicines in the field of medicine through the target deep learning framework, etc.

The following exemplarily illustrates the process of training the deep learning framework in specific application scenarios in combination with the specific application scenarios:

### Example 1:

In the present example, recognition of image semantics will be performed through the target deep learning framework.

Specifically, the training start condition will be determined according to the requirements of recognition of image semantics. In the present example, the training start condition is the preset number of the nodes in the idle state is greater than 5, and then the nodes in the idle state will be determined as the task nodes when the number of the nodes in the idle state in the current task node cluster is greater than or equal to 5.

Furthermore, the training of the deep learning framework for image semantic recognition will start at each task node. The training sample images of each task node may be acquired online or may also be read locally from the task node. The training sample images of each task node may be not completely the same. Unsupervised training methods will be used so that the frame parameters corresponding to the image semantics will be learned and obtained by the deep learning framework of each task node, and in the present embodiment, the average value of the frame parameters of all task nodes will be calculated at regular time intervals, and the deep learning framework in each task node will be synchronized according to the average value.

In the present embodiment, a semantic loss value of the last synchronized deep learning framework may be calculated according to a loss function. For example, a reference training image annotated with standard semantics may be input into the last synchronized deep learning framework to acquire reference semantics output by the last synchronized deep learning framework, and the semantic loss value of the reference semantics and the standard semantics may be calculated. And when the loss value is less than a preset threshold, the last synchronized deep learning framework may be considered to be the target deep learning framework that can be used to recognize the image semantics.

After the trained target deep learning framework is obtained, images, for which semantics is to be recognized, may be input into the deep learning framework, and the image semantics output by the target deep learning framework may be acquired.

### Example 2:

In the present example, determination of category labels of medicine categories will be performed through the target deep learning framework.

Specifically, the training start condition will be determined according to the medicine categories. In the present example, the training start condition is the preset number of the nodes in the idle state is greater than 20, and then the nodes in the idle state will be determined as the task nodes when the number of the nodes in the idle state in the current task node cluster is greater than or equal to 20.

Furthermore, the training of the deep learning framework for category labels of medicine categories will start at each task node. The training sample data of each task node may be acquired online or may also be read locally from the task node, wherein the training sample data may be information on the medicine ingredients, medicine manufacturer of various medicines, etc. The training sample images of each task node may be not completely the same. Unsupervised training methods will be used so that the frame parameters corresponding to the category recognition will be learned and obtained by the deep learning framework of each task node, and in the present embodiment, the average value of the frame parameters of all task nodes will be calculated at regular time intervals, and the deep learning framework in each task node will be synchronized according to the average value.

In the present embodiment, a category label loss value of the last synchronized deep learning framework may be calculated according to a loss function. For example, a reference medicine information annotated with standard category labels may be input into the last synchronized deep learning framework to acquire reference category labels output by the last synchronized deep learning framework, and the category label loss value of the reference category labels and the standard category labels may be calculated. And when the loss value is less than a preset threshold, the last synchronized deep learning framework may be considered to be the target deep learning framework that can be used to recognize the category labels of the medicines.

After the trained target deep learning framework is obtained, information on medicines, for which category labels is to be recognized, may be input into the deep learning framework, and the category labels output by the target deep learning framework may be acquired. As a result, training automation will be realized throughout the above-described training process, with no need for a user to monitor cluster resources and character status, etc., which reduces manpower costs for training and improves training efficiency.

In summary, according to the method for training the deep learning framework of the embodiment of the present disclosure, at least one task node in a current task node cluster is acquired, which meets a preset opening condition when a target task meets a training start condition, and then it is judged whether the number of the at least one task node is greater than a preset number, if the number of nodes is greater than the preset number, the deep learning framework of the target task is synchronously trained by the at least one task node according to sample data; at last a target deep learning framework synchronously trained by the task nodes is acquired when the target task meets a training completion condition. As a result, automatic and flexible training of the deep learning framework is realized, and under the premise of ensuring the training effect, the training efficiency is improved and the manpower cost is reduced.

It should be noted that in different application scenarios, the training start conditions corresponding to the target task and the preset opening conditions corresponding to the at least one task node are different. First, the training start conditions corresponding to the target task will be exemplified, and the description is as follows:

### Example 1:

In one embodiment of the present invention, in order to facilitate automated management of the training of the target task, four states of the task will be set. As shown in Fig. 2, the four states of the training of the task include: activated, waiting, running, finished. Refer to Fig. 2 for the transition relationship between them. The initial state of each task is "activated". When training is performed in the cluster resource, the state of the task is running. The running state means that the task is running normally. In the case that it is interrupted, preempted, fails due to machine failure or other reasons, it will switch to the waiting state. In the waiting state, when the cluster resource meets the relevant requirements for the start of training again, the training task will turn to the running state again. When the training of the whole task completes smoothly, it will turn to the finished state, which indicates that the training is completed.

Therefore, in the present embodiment, the target task meets the training start condition, and it is possible that the target task is started to the activated state, or the target task is in the waiting state.

In the present implementation, in order to ensure that each task node still retains the previous training results after entering the waiting state, during the training of the task, the framework parameters of the corresponding deep learning framework and the like will be saved at regular time intervals, so as to facilitate an automatic hot start of the next training from the last saved framework parameters in the waiting state when the machine resources are satisfied again and turn the training task to the running state again.

### Example 2:

In the present embodiment, an interface for artificially triggering the training start is provided. When a keyword for starting the training of the target task, which is contained in a voice message from a user, is received, or the user triggers a preset training start control, it is determined that the target task meets the training start condition.

### Example 3:

In the present example, in order not to hinder the progress of other tasks of high priority, only when there is no task of high level needed to be performed, the task meets the training start condition.

In the present embodiment, a priority level of a task to be executed in the current task node cluster will be monitored and it is determined that the target task meets the training start condition if the priority level is less than a preset level.

It is not difficult to understand that the cluster resources may carry a large number of training tasks, and the execution of the training of the target task requires a certain amount of resources. Therefore, in order to ensure that the target task can be executed, at least one task node in a current task node cluster will be acquired, that meets a preset opening condition when a target task meets a training start condition, wherein each node in the cluster may be understood as a machine resource, which can be used for the training of the deep learning framework according to the sample data.

Second, the preset opening conditions corresponding to at least one task node will be exemplified, and the exemplification is as follows:

### Example 1:

In the present example, the preset opening condition is the node state being the idle state.

In the present example, the node state of each node in the current task node cluster will be determined. The node state may include a task execution state and the idle state and the like. In the present embodiment, the node, the node state of which is the idle state, will be determined to be the task node that meets the preset opening condition.

In the present embodiment, a query request for the status bit may be sent to each node, and the node state of each node may be determined according to feedback information from each node.

### Example 2:

In the present example, the preset opening condition is the amount of idle resources being greater than a certain value.

In the present embodiment, the amount of idle resources, for example, the amount of idle memories, of each node in the current task node cluster will be determined. For example, when each node is also a sub-cluster, the amount of idle resources may be the number of byte points, which are idle in each node, etc.

Then a node, the amount of idle resources of which is greater than a preset threshold, will be determined as the task node that meets the preset opening condition, wherein the preset threshold can be determined according to the training amount of the target task.

In summary, in the method for training the deep learning framework of the embodiment of the present disclosure, the task node corresponding to the target task will be determined by flexibly using different methods according to the requirements of the scenarios, which further improves the automation of the training of the target task.

Since a data parallel strategy will be adopted in the self-supervised scheme, for a task, the more nodes used means the greater degree of parallelism and the shorter training time.

It should be noted that in supervised training, because when training a deep neural network, there is a training requirement to keep the identical distributed input of each layer of neural network in the course of the training of the deep neural network, so if the number of nodes is increased or decreased in the course of the training, fluctuations in statistics may be caused, which leads to unstable training and detrimental effects. However, in the above described several solutions of the supervised training the above described training requirements are global, and therefore the increasing or decreasing of the nodes will not influence the effect at all. And this is the theoretical guarantee of the flexible training module we designed

In one embodiment of the present invention, an automatic flexible pre-training framework is provided, which can make full use of the idle resources of the cluster to complete self-supervised training, which is a very time-consuming task. More importantly, the globalized requirements of the above training requirements enable the synchronization of the framework parameters of the training among multiple task nodes, which then ensures that the flexible training has no loss in effects. And the automatic detection and retrieval mechanism can save the investigator from checking the task status frequently, and free up manpower to do more meaningful things.

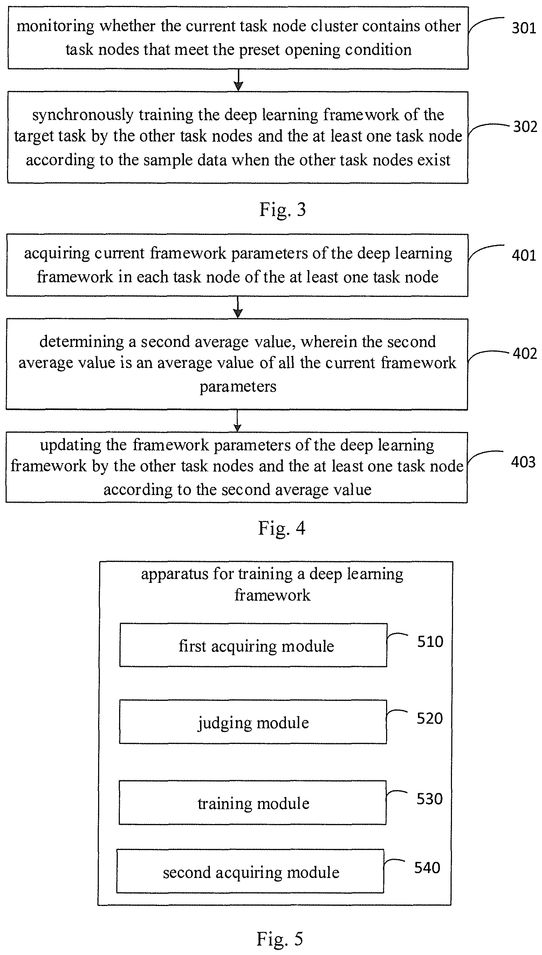

Specifically, Fig. 3 is a flowchart of a method for training a deep learning framework according to an embodiment of the present disclosure. As shown in Fig. 3, the method further includes before obtaining the training completion instruction of the task in the above step:
a step 301 of monitoring whether the current task node cluster contains other task nodes that meet the preset opening condition.

In the present embodiment, in the process of the training of the node, that is, when the task is in the training process, that is, when the task is in the running state, if it is found that the cluster has more idle nodes, it can also be directly "popped" out, so as to use more nodes to accelerate the training by making full use of cluster resources. Or when the task node enters the running state from the waiting state, it is monitored whether the current task node cluster contains other task nodes that meet the preset opening condition.
a step 302 of synchronously training the deep learning framework of the target task by the other task nodes and the at least one task node according to the sample data if the other task nodes exist.

In the present embodiment, if the other task nodes exist, the deep learning framework will be trained synchronously by the other task nodes and the at least one task node according to the sample data. For example, in a certain task, 4 task nodes were used for the training for the first time; and the task enters the waiting state because of being preempted; and when the training start condition of the target task was met again, there are 8 idle task nodes, and then we will automatically assign 8 task nodes to the task at this time, so as to achieve the purpose of flexibility.

In an embodiment of the present disclosure, the training will not be performed by the other task nodes from the initial state of the deep learning framework, but from a state, in which it has been trained by a task node. As shown in Fig. 4, the synchronously training the deep learning framework of the target task according to the other task nodes and the at least one task node includes:
a step 401 of acquiring current framework parameters of the deep learning framework in each task node of the at least one task node.

It is understandable that the frame parameters of the deep learning framework in each task node are the results of the current task node, which has trained.
a step 402 of determining a second average value, wherein the second average value is an average value of all the current framework parameters;
a step 403 of updating the framework parameters of the deep learning framework by the other task nodes and the at least one task node according to the second average value.

In the present embodiment, the second average value of the frame parameters of all task nodes will be calculated, and the frame parameters of the deep learning framework will be updated according to the second average value to obtain updated reference frame parameters, which contain the current training results of all the task nodes.

In the present embodiment, the deep learning framework of the target task will be trained according to the other task nodes and the at least one task node, which improves the training efficiency of the task nodes.

Of course, in one embodiment of the present disclosure, in order to reduce the occupancy rate of nodes, an upper limit of the other nodes may also be set according to the training amount of the target task. When the number of the other nodes is greater than the upper limit, the nodes of the number corresponding to the upper limit may be randomly selected from the task nodes that meet the preset condition, as the other nodes.

In summary, in the method for training the deep learning framework according to an embodiment of the present disclosure, an automatic flexible pre-training framework is used, which can make full use of the idle resources of the cluster to complete self-supervised training, which is a very time-consuming task, and improve the training efficiency while ensuring the training effects.

In order to implement the above embodiments, the present disclosure also proposes an apparatus for training a deep learning framework. Fig. 5 is a schematic structural diagram of the apparatus for training the deep learning framework according to one embodiment of the present disclosure. As shown in Fig. 5, the apparatus includes: a first acquiring module 510, a judging module 520, a training module 530, and a second acquiring module 540, wherein

the first acquiring module 510 is configured for acquiring at least one task node in a current task node cluster, that meets a preset opening condition when a target task meets a training start condition;
the judging module 520 is configured for judging whether the number of nodes of the at least one task node is greater than a preset number;
the training module 530 is configured for synchronously training the deep learning framework of the target task according to the at least one task node according to sample data when the number of nodes is greater than the preset number;
the second acquiring module 540 is configured for acquiring a target deep learning framework for synchronous training when the target task meets a training completion condition.

In one embodiment of the present disclosure, the first acquiring module 510 is specifically configured for:
determining a node status of each node in the current task node cluster;
determining a node, the node state of which is an idle state condition, as the at least one task node that meets the preset opening condition.

In one embodiment of the present disclosure, the first acquiring module 510 is specifically configured for:
determining the amount of idle resources of each node in the current task node cluster;
determining a node, the amount of idle resources of which is greater than a preset threshold condition, as the at least one task node that meets the preset opening condition.

In one embodiment of the present disclosure, the training module 530 is specifically configured for:
training the deep learning framework in each of the task nodes;
reading framework parameters of the deep learning framework in each of the task nodes in each period according to a preset period;
determining a first average value, wherein the first average value is an average value of the framework parameters of all the task nodes.

It should be noted that the foregoing explanation of the method for training the deep learning framework is also applicable to the apparatus for training the deep learning framework of the embodiment of the present disclosure, and the implementation principles of which are similar, and will not be repeated here.

In summary, according to the apparatus for training the deep learning framework of the embodiment of the present disclosure, at least one task node in a current task node cluster is acquired, which meets a preset opening condition when a target task meets a training start condition, and then it is judged whether the number of the at least one task node is greater than a preset number, if the number of nodes is greater than the preset number, the deep learning framework of the target task is synchronously trained by the at least one task node according to sample data; at last a target deep learning framework synchronously trained by the at least one task nodes is acquired when the target task meets a training completion condition. As a result, automatic and flexible training of the deep learning framework is realized, and under the premise of ensuring the training effect, the training efficiency is improved and the manpower cost is reduced.

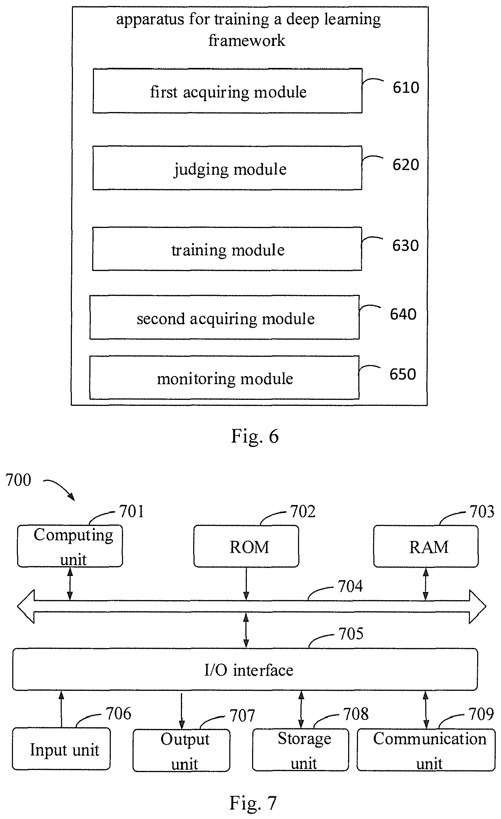

In one embodiment of the present disclosure, as shown in Fig. 6, the apparatus for training the deep learning framework includes: a first acquiring module 610, a judging module 620, a training module 630, a second acquiring module 640 and a monitoring module 650, wherein the functions of the first acquiring module 610, the judging module 620, the training module 630 and the second acquiring module 640 are the same as
those of the first acquiring module 510, the judging module 520, the training module 530, and the second acquiring module 540 in the above described embodiment, and will not repeated here.

In the present embodiment, the monitoring module 650 is configured for monitoring whether the current task node cluster contains other task nodes that meet the preset opening condition;
the training module 630 is further configured for synchronously training the deep learning framework of the target task by the other task nodes and the at least one task node according to the sample data if the other task nodes exist.

In one embodiment of the present disclosure, the training module 630 is specifically configured for:
acquiring current framework parameters of the deep learning framework in each task node of the at least one task node;
calculating a second average value of all the current framework parameters,
according to the second average value, which is an average value of all the current framework parameters;
updating the framework parameters of the deep learning framework by the other task nodes and the at least one task node according to the second average value.

In one embodiment of the present disclosure, the first acquiring module 510 is specifically configured for:
monitoring a priority level of a task to be executed in the current task node cluster;
determining that the target task meets the training start condition when the priority level is less than a preset level.

It should be noted that the foregoing explanation of the method for training the deep learning framework is also applicable to the apparatus for training the deep learning framework of the embodiment of the present disclosure, and the implementation principles of which are similar, and will not be repeated here.

In summary, in the apparatus for training the deep learning framework according to an embodiment of the present disclosure, an automatic flexible pre-training framework is used, which can make full use of the idle resources of the cluster to complete self-supervised training, which is a very time-consuming task, and improve the training efficiency while ensuring the training effects.

According to embodiments of the present disclosure, the present disclosure also provides an electronic device, a readable storage medium, and a computer program product.

Fig. 7 shows a schematic block diagram of an example electronic device 700 that can be used to implement embodiments of the present disclosure. An electronic device is intended to represent various forms of digital computers, such as a laptop computer, a desktop computer, a workstation, a personal digital assistant, a server, a blade server, a mainframe computer and other suitable computers. An electronic device can also represent various forms of mobile apparatuses, such as personal digital processing, cellular phone, smart phone, a wearable device and other similar computing apparatuses. The components shown herein, their connections and relationships and their functions are merely examples, and are not intended to limit the implementation of the present disclosure described and/or required herein.

As shown in Fig. 7, the device 700 includes a computing unit 701, which can perform various suitable actions and processing according to a computer program stored in a read-only memory (ROM) 702 or a computer program loaded from a storage unit 708 to a random access memory (RAM) 703. In the RAM 703, various programs and data required for operations of the device 700 may also be stored. The computing unit 701, the ROM 702 and the RAM 703 are connected to each other through a bus 704. An input/output (I/O) interface 705 is also connected to the bus 704.

Multiple components in the device 700 are connected to the I/O interface 705, including: an input unit 706, such as a keyboard, a mouse, etc.; an output unit 707, such as various types of displays, speakers, etc.; and the storage unit 708, such as a disk, an optical disc, etc.; and a communication unit 709, such as a network card, a modem, a wireless communication transceiver, etc. The communication unit 709 allows the device 700 to exchange information/data with other devices through a computer network such as the Internet and/or various telecommunication networks.

The computing unit 701 may be various general-purpose and/or special-purpose processing components with processing and computing capabilities. Some examples of the computing unit 701 include, but are not limited to, a central processing unit (CPU), a graphics processing unit (GPU), various dedicated artificial intelligence (AI) computing chips, various computing units that run machine learning model algorithms, a digital signal processing (DSP), and any suitable processor, controller, microcontroller, etc. The computing unit 701 executes the various methods and processes described above, such as the method for training the deep learning framework. For example, in some embodiments, the method for training the deep learning framework may be implemented as a computer software program, which is tangibly contained in a machine-readable medium, such as the storage unit 708. In some embodiments, part or all of the computer program may be loaded and/or installed onto the device 700 via the ROM 702 and/or the communication unit 709. When the computer program is loaded into the RAM 703 and executed by the computing unit 701, one or more steps of the method for training the deep learning framework described above can be executed. Alternatively, in other embodiments, the computing unit 701 may be configured to perform the method for training the deep learning framework in any other suitable manner (for example, by means of firmware).

The above various embodiments of the systems and technologies described herein can be implemented in a digital electronic circuit system, an integrated circuit system, a field programmable gate array (FPGA), an application specific integrated circuit (ASIC), an application-specific standard product (ASSP), a system of a system on chip (SOC), a load programmable logic device (CPLD), a computer hardware, firmware, software, and/or a combination thereof. These various embodiments may include: being implemented in one or more computer programs, which may be executed and/or interpreted on a programmable system including at least one programmable processor, the programmable processor may be a dedicated or general-purpose programmable processor that can receive data and instructions from a storage system, at least one input device and at least one output device, and transmit data and instructions to the storage system, the at least one input device and the at least one output device.

The program codes used to implement the method of the present disclosure can be written in any combination of one or more programming languages. These program codes can be provided to a processor or controller of a general-purpose computer, a special-purpose computer or other programmable data processing devices, so that when the program codes are executed by the processor or controller, the functions/operations specified in the flowcharts and/or block diagrams are implemented. The program codes can be executed entirely on a machine, partly executed on a machine, partly executed on a machine and partly executed on a remote machine as an independent software package, or entirely executed on a remote machine or a server.

In the context of the present disclosure, a machine-readable medium may be a tangible medium, which may contain or store a program for use by an instruction execution system, an apparatus or a device or for use in combination with an instruction execution system, an apparatus or a device. The machine-readable medium may be a machine-readable signal medium or a machine-readable storage medium. The machine-readable medium may include, but is not limited to, an electronic, magnetic, optical, electromagnetic, infrared or semiconductor system, an apparatus or a device or any suitable combination of the foregoing. More specific examples of the machine-readable storage medium may include electrical connection based on one or more wires, a portable computer disk, a hard disk, a random access memory (RAM), a read-only memory (ROM), an erasable programmable read-only memory (EPROM or flash memory), an optical fiber, a portable compact disk read-only memory (CD-ROM), an optical storage device, a magnetic storage device or any suitable combination of the foregoing.

In order to provide interaction with a user, the systems and technologies described here can be implemented on a computer, which has: a display apparatus for displaying information to the user (for example, a CRT (cathode ray tube) or LCD (liquid crystal display) monitor); and a keyboard and pointing apparatus (for example, a mouse or a trackball), through which the user can provide input to the computer. Other types of apparatuses can also be used to provide interaction with the user; for example, the feedback provided to the user can be any form of sensory feedback (for example, visual feedback, auditory feedback or tactile feedback); and input from the user can be received in any form (including acoustic input, voice input or tactile input).

The systems and technologies described here can be implemented in a computing system that includes back-end components (for example, as a data server), or a computing system that includes middleware components (for example, an application server), or a computing system that includes front-end components (for example, a user computer with a graphical user interface or a web browser, through which the user can interact with the embodiments of the systems and technologies described herein), or a computing system that includes any combination of such background components, middleware Components or front-end components. The components of the system can be connected to each other through any form or medium of digital data communication (for example, a communication network). Examples of the communication network include: a local area network (LAN), a wide area network (WAN), the Internet, and a blockchain network.

The computer system may include a client and a server. The client and the server are generally far away from each other and usually interact through a communication network. The relationship between the client and the server will be generated by a computer program that runs on a corresponding computer and has a client-server relationship with each other. The server may be a cloud server, also known as a cloud computing server or a cloud host, which is a host product in a cloud computing service system to solve the defect of the existed shortcomings of difficult management and weak business scalability in the traditional physical host and VPS service ("Virtual Private Server", or "VPS" for short). The server may also be a server of a distributed system, or a server combined with a blockchain.

The server may also be a server of a distributed system, or a server combined with a blockchain.

It should be understood that the various forms of flows shown above can be used to reorder, add or delete steps. For example, the respective steps described in the present disclosure may be executed in parallel, or also may be executed sequentially, or also may be executed in a different order, as long as the desired result of the technical solution disclosed in the present disclosure can be achieved, and will be not limited herein.

The foregoing specific embodiments do not constitute limitation on the protection scope of the present disclosure. Those skilled in the art should understand that various modifications, combinations, sub-combinations and substitutions can be made according to design requirements and other factors. Any modification, equivalent replacement and improvement and the like made within the principle of the present disclosure shall be included in the protection scope of the present disclosure.

## Claims

1. A method for training a deep learning framework, comprising:
acquiring (101) at least one task node in a current task node cluster, that meets a preset opening condition when a target task meets a training start condition;
judging (102) whether a number of nodes of the at least one task node is greater than a preset number;
synchronously training (103) the deep learning framework of the target task by the at least one task node according to sample data when the number of nodes is greater than the preset number; and
acquiring (104) a synchronously trained target deep learning framework when the target task meets a training completion condition.

2. The method according to claim 1, wherein the acquiring (101) the at least one task node in the current task node cluster, that meets the preset opening condition, comprises:
determining a node state of each node in the current task node cluster; and
determining a node, the node state of which is an idle state condition, as the at least one task node that meets the preset opening condition.

3. The method according to claim 1 or 2, wherein the acquiring (101) the at least one task node in the current task node cluster, that meets the preset opening condition, comprises:
determining an amount of idle resources of each node in the current task node cluster; and
determining a node, the amount of idle resources of which is greater than a preset threshold condition, as the at least one task node that meets the preset opening condition.

4. The method according to any one of claims 1 to 3, wherein the synchronously training (103) the deep learning framework of the target task by the at least one task node according to the sample data comprises:
training the deep learning framework in each task node;
reading framework parameters of the deep learning framework in each task node in each period according to a preset period;
determining a first average value, wherein the first average value is an average value of the framework parameters of all task nodes; and
synchronizing the deep learning framework in each task node according to the first average value.

5. The method according to any one of claims 1 to 4, wherein the synchronously training (103) the deep learning framework of the target task by the at least one task node according to the sample data comprises:
monitoring (301) whether the current task node cluster contains other task nodes that meet the preset opening condition; and
synchronously (302) training the deep learning framework of the target task by the other task nodes and the at least one task node according to the sample data when the other task nodes exist.

6. The method according to claim 5, wherein the synchronously training (302) the deep learning framework of the target task by the other task nodes and the at least one task node according to the sample data comprises:
acquiring (401) current framework parameters of the deep learning framework in each task node of the at least one task node;
determining (402) a second average value, wherein the second average value is an average value of all the current framework parameters; and
updating (403) the framework parameters of the deep learning framework by the other task nodes and the at least one task node according to the second average value.

7. The method according to any one of claims 1 to 6, wherein when the target task meets the training start condition, the method comprises:
monitoring a priority level of a task to be executed in the current task node cluster; and
determining that the target task meets the training start condition when the priority level is less than a preset level.

8. An apparatus for training a deep learning framework, comprising:
a first acquiring module (510), configured to acquire at least one task node in a current task node cluster, that meets a preset opening condition when a target task meets a training start condition;
a judging module (520), configured to judge whether a number of nodes of the at least one task node is greater than a preset number;
a training module (530), configured to synchronously train the deep learning framework of the target task by the at least one task node according to sample data when the number of nodes is greater than the preset number; and
a second acquiring module (540), configured to acquire a synchronously trained target deep learning framework when the target task meets a training completion condition.

9. The apparatus according to claim 8, wherein the first acquiring module (510) is configured to:
determine a node state of each node in the current task node cluster; and
determine a node, the node state of which is an idle state condition, as the at least one task node that meets the preset opening condition, or
wherein the first acquiring module (510) is configured to:
determine an amount of idle resources of each node in the current task node cluster; and
determine a node, the amount of idle resources of which is greater than a preset threshold condition, as the at least one task node that meets the preset opening condition.

10. The apparatus according to claim 8 or 9, wherein the training module (530) is configured to:
train the deep learning framework in each task node;
read framework parameters of the deep learning framework in each node in each period according to a preset period;
determine a first average value, wherein the first average value is an average value of the framework parameters of all task nodes.

11. The apparatus according to any one of claims 8 to 10, further comprising:
a monitoring module (650), configured to monitor whether the current task node cluster contains other task nodes that meet the preset opening condition;
the training module (630) is further configured to synchronously train the deep learning framework of the target task by the other task nodes and the at least one task node according to the sample data when the other task nodes exist.

12. The apparatus according to claim 11, wherein the training module (630) is configured to:
acquire current framework parameters of the deep learning framework in each task node of the at least one task node;
determine a second average value, wherein the second average value is an average value of all the current framework parameters; and
update the framework parameters of the deep learning framework by the other task nodes and the at least one task node according to the second average value.

13. An electronic device, comprising:
at least one processor; and
a memory communicatively connected with the at least one processor; wherein,
instructions executable by the at least one processor are stored in the memory, and the instructions are executed by the at least one processor, so that the at least one processor executes the method for training the deep learning framework according to any of claims 1-7.

14. A non-transitory computer-readable storage medium, in which computer instructions are stored, wherein the computer instructions are configured to cause a computer execute the method for training the deep learning framework according to any of claims 1-7.

15. A computer program product, comprising a computer program that, when executed by a processor, implements the method for training a deep learning framework according to any of claims 1-7.
